Europäisches Patentamt

European Patent Office

Office européen des brevets

(11) Publication number: **0 345 251 B1**

# EUROPEAN PATENT SPECIFICATION

(45) Date of publication of patent specification: **11.11.92**

(51) Int. Cl.⁵: **A61K 31/73**, //(A61K31/73, 31:475),(A61K31/73,31:65), (A61K31/73,31:135), (A61K31/73,31:40)

(21) Application number: **87905260.3**

(22) Date of filing: **21.08.87**

(86) International application number: **PCT/GB87/00589**

(87) International publication number: **WO 88/01171 (25.02.88 88/05)**

(54) **PHARMACEUTICAL COMPOSITIONS FOR THE TREATMENT OF CANCERS.**

(30) Priority: **21.08.86 GB 8620361**

(43) Date of publication of application: **13.12.89 Bulletin 89/50**

(45) Publication of the grant of the patent: **11.11.92 Bulletin 92/46**

(84) Designated Contracting States: **AT BE CH DE FR GB IT LI LU NL SE**

(56) References cited: **WO-A-80/01875**

(73) Proprietor: **FIDIA S.p.A. Via Ponte della Fabbrica 3-A I-35031 Abano Terme (Padova)(IT)**

(72) Inventor: **HELLMANN, Kurt Windleshaw House, Withyham Near Hartfield East Sussex TN7 4DB(GB)**

(74) Representative: **Kinzebach, Werner, Dr. et al Patentanwälte Reitstötter, Kinzebach und Partner Sternwartstrasse 4 Postfach 86 06 49 W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention is concerned with new pharmaceutical compositions for the treatment and amelioration of various types of cancer.

A large number of compounds are already known which can be used with considerable beneficial effect for the treatment cancers. However, almost all compounds at present used for the treatment of cancer are themselves extremely toxic and can give rise to highly undesirable haematological, gastro-intestinal, neurological and other side-effects, especially when administered for long periods of time such as are usually necessary in the treatment of cancers.

Gangliosides are glycosphingolipids which occur in high concentration in the central nervous system. They consist of fatty acids, sphingosine, hexoses, galactosamine and sialic acid. A ganglioside preparation is commercially available under the name "Cronassial®", the composition of which has been found to be as follows:

| Monosialotetraesosil ganglioside | (GM- 1) | 21% |
| disialotetraesosil ganglioside | (GD-1a) | 40% |
| disialotetraesosil ganglioside | (GD-1b) | 16% |
| trisialotetraesosil ganglioside | (GT-1b) | 19% |

Cronassial® has been successfully used for the treatment of neurological and associated diseases.

In view of the fact that many of the highly undesirable side effects associated with the therapy of cancers are neurological, we have investigated the possibility of simultaneously carrying out treatments with several different, known compounds which have already been used for the treatment of cancers, together with a treatment with Cronassial®.

A number of anti-cancer compounds which we have investigated, include mitosene derivatives, such as mitomycin C and porfiromycin; anthracyclines, such as adriamycin; mitozantrone and vincristine.

In an initial series of experiments, these anti-cancer drugs were tested with and without gangliosides on the following tumours; sarcoma S180, melanoma B16, leukaemia L 1210 and Lewis lung tumour 3LL. Cronassial® was also administered separately. We found that Cronassial® had a positive effect on the activity and toxicity of adriamycin, vincristine, mitomycin C and mitozantrone. Because Cronassial® reduces the toxicity of the anti-cancer drugs tested, this means that it is possible to administer higher dosages of the drugs to the patients without increasing the risk of toxic side effects.

In a further series of experiments, we prepared mixtures of Cronassial® with vincristine, adriamycin, mitomycin C and mitozantrone with the object of simplifying administration.

However, a further series of experiments on the above-mentioned tumours showed that the positive effects achieved when administering Cronassial® in admixture with vincristine, adriamycin, mitomycin C and mitozantrone were significantly better than the results achieved by administering Cronassial® separately from vincristine, adriamycin, mitomycin C and mitozantrone.

Vincristine is a colourless crystalline solid which dissolves to give a colourless solution, whereas mitozantrone is a blue-black solid which dissolves to give a dark blue solution. When Cronassial® is added to a solution of mitozantrone, there is a considerable change of the colour of the solution to a blue/green colour. Further experiments demonstrated that the depth of colour of a solution of mitozantrone is not dependent upon the pH value of solution.

Consequently, it would appear that a chemical reaction or change takes place when Cronassial is added to a solution of mitozantrone and it would appear, therefore, that it is this reaction or change which brings about the significant improvement of the beneficial results which are achieved by the administration of a mixture of Cronassial® with mitoxantrone.

Similar experiments with adriamycin showed a colour shift from red to orange/red and with mitomycin C showed a colour shift from violet to blue.

Because vincristine is colourless in solution, no visible changes were observed when Cronassial® was added to a solution of vincristine but, since such a mixture also showed a similar significant improvement of the beneficial results, it is reasonable to assume that, here again, a chemical reaction or change takes place.

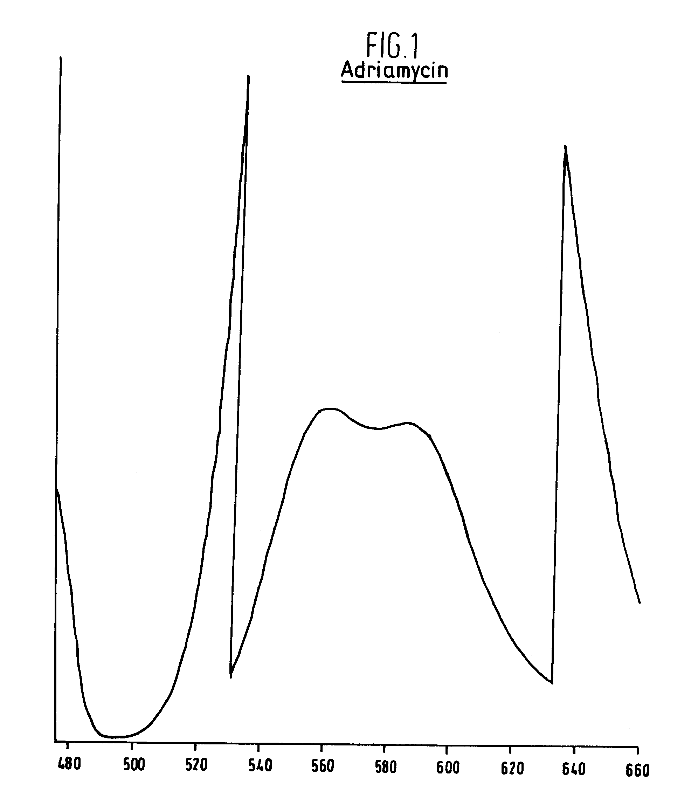

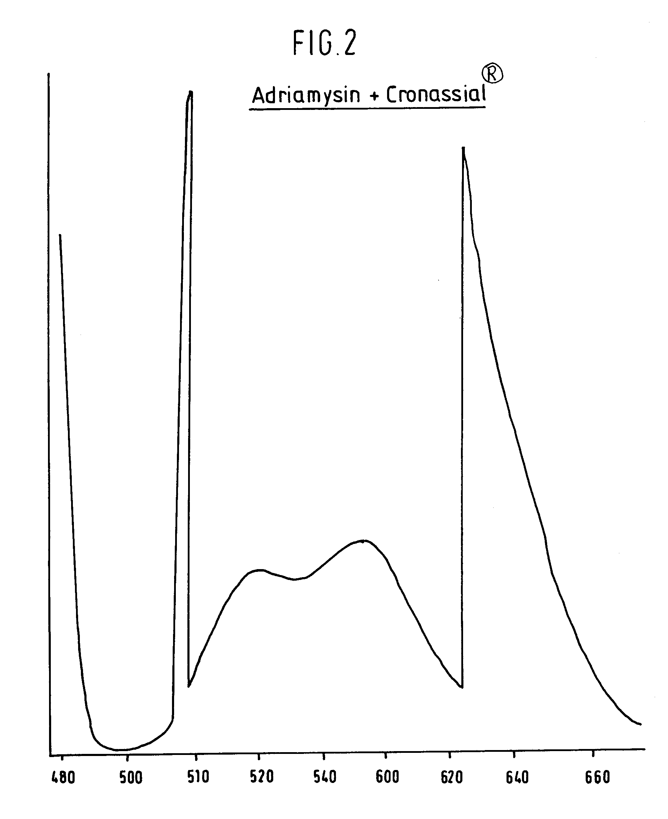

Although it appears that a chemical reaction or change takes place between the anti-cancer drugs tested and Cronassial®, we have not yet been able to ascertain whether some or all of the components of Cronassial® participate in this chemical reaction or change. Figures 1 and 2 of the accompanying drawings show the fluorescent spectrum of adriamycin alone and after mixing with Cronassial®. The considerable differences between the two spectra provide evidence that a reaction appears to have taken place between the two components.

Therefore, according to the present invention, there is provided a composition for the treatment of cancers, which comprises at least one anti-cancer drug selected from the mitosene derivatives, such as mitomycin C and porfiromycin; the anthracyclines, such as adriamycin; Vinca alkaloids, such as vincristine; and anthracenediones, such as mitozantrone, in admixture with at least one ganglioside.

The ganglioside used can be, for example, in the form of the above-described "Cronassial®".

Since the anti-cancer drugs present in the compositions according to the present invention are administered intravenously in isotonic solution, the compositions according to the present invention are also preferably in the form of an isotonic solution suitable for intravenous administration. In other words, the compositions according to the present invention are prepared and administered in the same way as the anti-cancer drugs present therein.

The weight ratio of anti-cancer drug to ganglioside in the compositions according to the present invention can be 1:1000, preferably 1:200 and most preferably 1:40.

Quite apart from a marked reduction in the toxicity of the known anti-cancer drugs, the compositions avoid variations in the amounts of drug complex administered and also greatly increase the up-take after administration. This means that the compositions according to the present invention result in an amelioration of the undesired side effects but also enable larger doses of anti-cancer drugs to be administered without the danger of increasing the undesired side-effects.

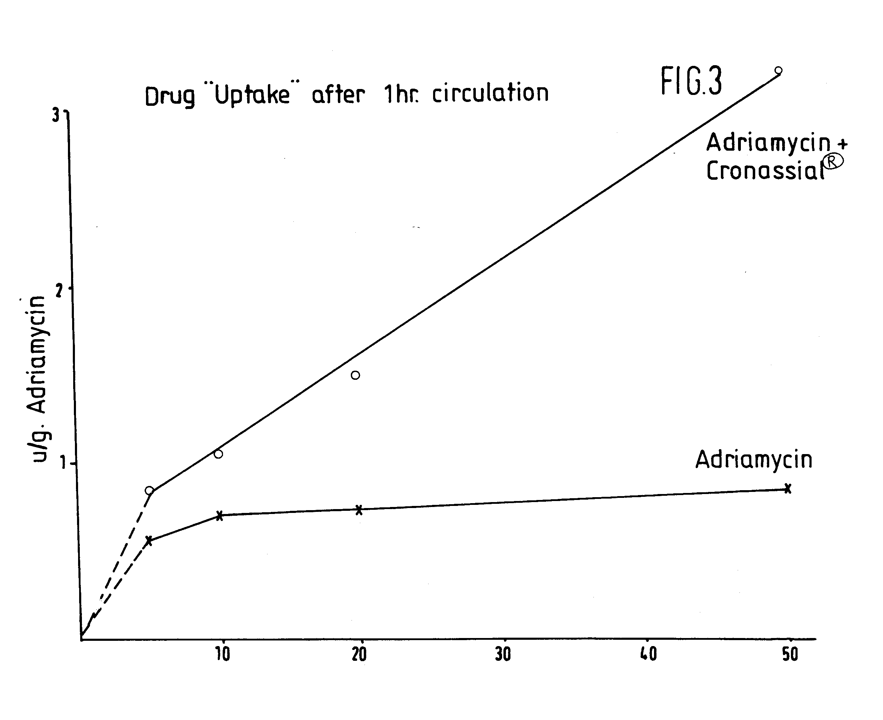

This is clearly shown by Figure 3 of the accompanying drawings, which demonstrates the take up of adriamycin when administered alone and when administered together with Cronassial®. The results given in Figure 3 were obtained by treating Chinese hamster ovary cells for one hour and clearly demonstrate that the adriamycin/Cronassial® complex behaves differently in solution to adriamycin alone.

The following is a survey of the experiments which have been carried out:

## MATERIALS AND METHODS

### ANIMALS

Toxicity acute and chronic. Male Swiss Schneider mice with a body weight of 25-30g were used for these experiments. Mice were usually kept in groups of not more than 8 in standard cages. Food and water were supplied ad libidum. The animals were kept in experimental rooms under closely controlled conditions. They were weighed daily. For acute vincristine toxicity in chicks, 48 hour old SPF chicks were used.

Antitumour effects. Male Swiss Schneider mice of 27g body weight were used for the sarcoma S180 experiments: female $C_{57}$Bl mice of 20g body weight were used for the B16 melanoma experiments; male $BDF_1$ mice of 30g body weight were used for the L1210 leukaemia experiments; and $C_{57}$Bl mice of 20g body weight were used for the Lewis lung experiments.

### TUMOURS

Sarcoma S180. This tumour has been transplanted in the same strain of mouse for more than 10 years.

Transplantation was done by subcutaneous inoculation of 0.1 ml of a tumour mash made by finely mincing viable tumour tissue and passing it repeatedly through a 26 gauge needle into a sterile Petri dish. 0.1 ml of penicillin (20,000 m/ml) and streptomycin (20,000 m/ml) were added to the mash, as well as neomycin (5 mgs).

Melanoma B16. This tumour was prepared for inoculation in a manner identical to that used for the S180 sarcoma.

L1210. Spleens were removed from animals 7 days after inoculation of L1210 cells and these spleens were finely minced with 1:100 isotonic saline. 0.1 ml of this spleen and leukaemia L1210 cell suspension was then injected subcutaneously into the flank of each $BDF_1$.

Lewis lung (3LL). This tumour was prepared for inoculation in a manner identical to that used for the S180 sarcoma.

### ACTIVE MATERIALS

Vincristine. A standard vial of 'Oncovin' (Eli Lilly Co.) containing 1 mg of vincristine was used. It was made up with the appropriate volume of diluting fluid to give the final concentration required to inject 0.1 ml/10 g body weight.

Cronassial®. A solution of this substance, which is a mixture of four gangliosides, was prepared by the addition of a volume of sterile distilled water sufficient to give a concentration of 200 mg/kg in a volume of 0.2 ml. This dose was given to mice of approximately 20g.

Mitozantrone was made up in an appropriate manner. It was given in a volume of 0.2ml when the injections were given intraperitoneally but when given by another route, the volumes given were indicated in the appropriate Table.

RESULTS

Acute toxicity

Large doses of vincristine (i.e. 3.0 mg/kg) given as one intravenous injection to Swiss Schneider mice proved to be fatal for 75% by day 30 (Table 1).

The same treatment but with 200 mg/kg Cronassial® given 6 hours before the vincristine resulted in 87.5% deaths by day 30.

It was apparent that Cronassial®, under these conditions, gave no protection against the acute toxicity of vincristine.

Acute toxicity experiments using 48 hour old chicks showed that, with high doses of vincristine (6 mg/kg), no acute neurotoxic signs were seen during the first 4 hours in 3/4 chicks but one chick became ataxic and one had preterminal convulsions at 24 hours. All the chicks were dead at 24 hours. In contrast,of those chicks which received 200 mg/kg Cronassial® at the same time as the vincristine, only 2/5 had died at 24 hours but only 1 remained alive at 28 hours.

With 4 mg/kg vincristine, 4/5 chicks survived for 24 hours but only 1/5 survived for 28 hours. The chicks which additionally, received 200 mg/kg Cronassial® survived better with 3/5 surviving to 28 hours.

With 2 mg/kg vincristine, there was little toxicity and this was not changed when Cronassial® was also given.

Therefore, it appears that acute lethal vincristine toxicity can be reduced by Cronassial® in large doses. Since even the largest doses of vincristine produced no neurotoxicity, it was not possible to judge whether Cronassial® affected this in any way.

Chronic Toxicity

Giving daily injections of vincristine of 0.8 mg/kg for 5 days was close to the $LD_{50}$. By giving 200 mg/kg Cronassial® at the same time as the vincristine, the number of survivors at 30 days was increased to 100% Almost identical results were obtained by using 1 mg/kg vincristine, even though the drugs were administered for only 4 days. However the total dosage of vincristine administered in the two experiments (4 mg/kg) was identical. Increasing the cumulative dose of vincristine from 4 to 5 mg/kg increased the mortality of the mice to 100%. With this overwhelming toxicity, there was little or no protection offered by the Cronassial®.

The results obtained are summarised in the following Table 1:-

4

TABLE 1

TOXICITY REDUCTION OF ANTITUMOUR DRUGS – CRONASSIAL® & VINCRISTINE

| TOXICITY | HOST | DRUG | DOSE mg/kg | DAYS | ROUTE | TIMING | SURVIVORS No.at 30 d | SURVIVAL % at 30 d |
|---|---|---|---|---|---|---|---|---|
| Chronic | SN | VCR | 0.8 | 1-5 | ip | | 5/8 | 62.5 |
| Chronic | SN | (VCR<br>(CRON | 0.8<br>200 | 1-5<br>1-5 | ip<br>ip | 6hrs before VCR | 7/7 | 100 |
| Chronic | SN | VCR | 1.0 | 1-4 | ip | | 3/8 | 37.5 |
| Chronic | SN | (VCR<br>(CRON | 1.0<br>200 | 1-4<br>1-4 | ip<br>ip | 6hrs before VCR | 6/8 | 75 |
| Chronic | SN | VCR | 1.0 | 1-5 | ip | | 0/6 | 0 |
| Chronic | SN | (VCR<br>(CRON | 1.0<br>200 | 1-5<br>1-5 | ip<br>ip | 6hrs before VCR | 1/5 | 20 |
| Acute | SN | VCR | 3.0 | 1 | iv | | 2/8 | 25 |
| Acute | SN | (VCR<br>(CRON | 3.0<br>200 | 1<br>1 | iv<br>ip | 6hrs before VCR | 1/8 | 12.5 |

CONCLUSION: (1) CRONASSIAL® DOES NOT REDUCE ACUTE VINCRISTINE TOXICITY.

(2) CRONASSIAL® REDUCES CHRONIC VINCRISTINE TOXICITY.

Antitumour activity with and without Cronassial®

Vincristine with and without Cronassial®, when tested against a panel of tumours consisting of S180, B16 and L1210, showed that Cronassial® did not interfere with any antitumour activity that vincristine might

5

have had as shown by inhibition of tumour growth or increase in mean survival time.

On the contrary, the results obtained against leukaemia L1210 show that the toxic deaths due to vincristine alone can be reduced, when given with Cronassial®, and that, therefore, the vincristine effectiveness against L1210 is enhanced with a mean survival time of 10.3 days compared with 7.8 days for vincristine alone. This difference is statistically significant.

The results obtained are summarised in the following Tables 2 and 3:-

## TABLE 2
### EFFECT OF CRONASSIAL® ON VINCRISTINE ANTITUMOUR ACTIVITY

| TUMOUR | HOST | DRUG | DOSE mg/kg | DAYS | ROUTE | TIMING | TUMOUR WEIGHTS MEAN (g) |
|---|---|---|---|---|---|---|---|
| S180 | SN | VCR | 0.5 | 1-6 | ip | | 0.64 |
| S180 | SN | (VCR<br>(CRON | 0.5<br>200 | 1-6<br>1-6 | ip<br>ip | 6hrs before VCR | 0.69 |
| S180 | SN | CMC-controls | | | | | 1.05 |
| B16 | C5781 | VCR | 0.5 | 1-4,7-11 | ip | | 1.46 |
| B16 | C5781 | (VCR<br>(CRON | 0.5<br>200 | 1-4,7-11 | ip | 6hrs before VCR | 1.66 |
| B16 | C5781 | CMC-controls | | | | | 2.33 |
| | | | | | | | Mean survival time (days) |
| L1210 | BDF$_1$ | VCR | 1.0 | 1-3 | ip | | 7.8 |
| L1210 | BDF$_1$ | (VCR<br>(CRON | 1.0<br>200 | 1-3<br>1-3 | ip<br>ip | same time as VCR | 10.3 |
| L1210 | BDF$_1$ | CMC-controls | | | | | 8.0 |

CONCLUSION: CRONASSIAL® DOES NOT REDUCE VINCRISTINE ACTIVITY

EP 0 345 251 B1

TABLE 3

TOXICITY REDUCTION – CRONASSIAL® & VINCRISTINE

| TOXICITY | HOST | DRUG | DOSE mg/kg | DAYS | ROUTE | TIMING | SURVIVORS AT | | |
|---|---|---|---|---|---|---|---|---|---|
| | | | | | | | 24hrs | 28hrs | 52hrs |
| Acute | SPF 48hr chick | VCR | 6.0 | 1 | ip | | 0/4 | - | - |
| Acute | SPF 48hr chick | (VCR (CRON | 6.0 200 | 1 | ip | at same time | 3/5 | 1/5 | 0/5 |
| Acute | SPF 48hr chick | VCR | 4.0 | 1 | ip | | 4/5 | 1/5 | 1/5 |
| Acute | SPF 48hr chick | (VCR (CRON | 4.0 200 | 1 | ip | at same time | 5/5 | 3/5 | 1/5 |
| Acute | SPF 48hr chick | VCR | 2.0 | 1 | ip | | 2/2 | 2/2 | 1/2 |
| Acute | SPF 48hr chick | (VCR (CRON | 2.0 200 | 1 | ip | at same time | 2/2 | 1/2 | 0/2 |

CONCLUSION: CRONASSIAL® APPEARS TO PROTECT AGAINST ACUTE LETHAL VINCRISTINE TOXICITY IN 48 HOURS OLD CHICKS.

Toxicity reduction of mitozantrone

The following Table 4 clearly shows that it is possible to obtain a toxicity reduction of mitozantrone by Cronassial® but that at least 200 mg/kg x 2 are necessary to achieve a definite improvement in survival and

8

the following Table 5 shows the results obtained from a series of experiments to determine the most effective time to give a combination of Cronassial® and mitozantone.

Antitumour activity of mitozantrone with and without Cronassial®

L1210

From the following Table 5, it can also be seen that there is consistent improvement of the results obtainable with mitozantrone against L1210 when Cronassial® is given together with the mitozantrone. This is not a synergistic effect but one which, by reducing the toxicity of mitozantrone, permits high doses of mitozantrone to be given against the L1210.

Lewis lung carcinoma (3LL)

This tumour metastasizes to the lungs when implanted into the flanks of mice. When treated for 5 days between day 5 and 9 after implantation with 3.0 mg/kg mitozantrone, 7/8 mice had died by day 14 so that no assessment could be made of the effect of mitozantrone on the primary or secondary tumours.

On the other hand, a combination of mitozantrone and Cronassial® showed that at day 22 the mean primary tumour weight had been reduced from 4.0 g to 1.47 g. and the number of secondaries had been reduced from a mean of 75.1 to 6.1 giving a highly significant T/C of 0.082.

The animals which had been treated with Cronassial® only, showed no difference in number of secondaries or in weight of primary tumours from the control animals treated with CMC.

TABLE 4

MINIMUM DOSE OF CRONASSIAL® REQUIRED TO REDUCE MITOZANTRONE TOXICITY

| TOXICITY | HOST | DRUG | DOSE | DAYS | ROUTE | TIMING | SURVIVORS No. at 30d | SURVIVAL (mean days) |
|----------|------|------|------|------|-------|--------|----------------------|----------------------|
| CHRONIC | SN | MTZ | 5 | 1,2 | ip | | 0/6 | 9 |
| CHRONIC | SN | (MTZ (CRON | 5 200 | 1,2 | ip | same time as MTZ | 2/6 | 31 |
| CHRONIC | SN | MTZ | 5 | 1,2 | ip | | 0/6 | 11 |
| CHRONIC | SN | (MTZ (CRON | 5 200 | 1,2 | ip | same time as MTZ | 1/6 | 14 |
| CHRONIC | SN | (MTZ (CRON | 5 100 | 1,2 | ip | same time as MTZ | 0/6 | 13 |
| CHRONIC | SN | (MTZ (CRON | 5 50 | 1,2 | ip | same time as MTZ | 0/6 | 11 |

CONCLUSION:   200mg/kg x 2 IS THE MINIMUM DOSE OF CRONASSIAL®

REQUIRED TO REDUCE MITOZANTRONE TOXICITY

## TABLE 5

MOST EFFECTIVE TIME AT WHICH TO GIVE A COMBINATION OF CRONASSIAL® AND MITOZANTRONE

| TUMOUR | HOST | DRUG | DOSE mg/kg | DAYS | ROUTE | TIMING | SURVIVORS No. at 60 d | SURVIVAL (mean days) |
|--------|------|------|-----------|------|-------|--------|----------------------|---------------------|
| L1210 | BDF$_1$ | MTZ | 5 | 0.1.2 | ip | | 1/8 | 6.14 |
| L1210 | BDF$_1$ | (MTZ (CRON | 5) 200) | 0.1.2. | ip | CR same as MTZ | 2/8 | 14.1 |
| L1210 | BDF$_1$ | MTZ | 5 | 1.2.3 | ip | | 2/8 | 17.1 |
| L1210 | BDF$_1$ | (MTZ (CRON | 5) 200) | 1.2.3 | ip | CR same as MTZ | 7/8 | >60 |
| L1210 | DBA | MTZ | 5 | 2.3.4 | ip | | 0/6 | 16.8 |
| L1210 | DBA | (MTZ (CRON | 5) 200) | 2.3.4 | ip | CR same as MTZ | 0/6 | 27.3 |
| L1210 | DBA | CMC-controls | | | ip | | 0/6 | 7.2 |
| L1210 | BDF$_1$ | MTZ | 5 | 3.4.5 | ip | | 0/8 | 25.2 |
| L1210 | BDF$_1$ | (MTZ (CRON | 5) 200) | 3.4.5 | ip | CR same as MTZ | 4/8 | 42.4 |
| L1210 | BDF$_1$ | CMC - controls | | | ip | | 0/8 | 8.7 |

Cronassial alone as control in all above experiments :            0/8            8.5

CONCLUSION:    CRONASSIAL® IS EFFECTIVE IN PROTECTING AGAINST MITAZANTRONE TOXICITY.

EP 0 345 251 B1

TABLE 6

CHANGED PROTECTION BY CRONASSIAL® OF MITOZANTRONE TOXICITY WITH S180

| TOXICITY | HOST | DRUG | DOSE mg/kg | DAYS | ROUTE | TIMING | SURVIVORS No. at 30d | SURVIVAL (mean days) |
|---|---|---|---|---|---|---|---|---|
| CHRONIC | SN (+S180) | MTZ | 5 | 3.4.5 | ip | | 0/8 | 8.8 |
| CHRONIC | SN (+S180) | (MTZ (CRON | 5 200 | 3.4.5 | ip | CR same time MTZ | | 20 |

CONCLUSION : THE PRESENCE OF S180 DOES NOT INTERFERE WITH THE

REDUCTION OF MITOZANTRONE TOXICITY WHICH

CRONASSIAL® PRODUCES.

Analysis of mechanism of toxicity reduction by Cronassial®

All the tests done show that vincristine and mitozantrone toxicity is clearly reduced by Cronassial®.

In trying to analyze the mechanism of toxicity protection by Cronassial®, the first step is a clear understanding of the toxicity induced by the drugs which have been affected by Cronassial®.

The three major systems affected by vincristine are haematological, gastro-intestinal and, in some species, neurological. It is extremely difficult to reproduce the neurotoxicity seen in man in any other species, apart from the cat and chicken, and it is unlikely, therefore, that any reduction of vincristine neurotoxicity by Cronassial® would have been seen in our experiments.

However, since there was little effect on acute toxicity in mice but clear evidence on chronic toxicity, it is likely that this could reflect what is seen in man, since it is rare for vincristine to produce acute toxic effects on the CNS or ANS, whereas the commonly encountered neurotoxicity in the clinic comes from chronic treatment.

On the time scale, therefore, the reduction of vincristine toxicity by Cronassial® which we have observed matches that observed in the clinic.

With regard to mitozantrone it would seem unlikely from the results given in the following Tables 7, 8 and 9 that the animals which did not survive had died a haematological death and, therefore, it is unlikely that protection of this system, even if it occurred, played any part in the toxicity reduction of mitozantrone by Cronassial®.

TABLE 7

| MEAN VALUES OF INDIVIDUAL ANIMALS RBC x $10^{12}$/l | | | | | | |
|---|---|---|---|---|---|---|
| | N | Day 5. | N | Day 7 | N | Day 8 |
| CONTROLS | 2 | 7.9 | | ND | 2 | 7.2 |
| MITOZANTRONE | 3 | 7.3 | 4 | 7.0 | 4 | 7.1 |
| MOTOZANTRONE & CRONASSIAL® | 4 | 7.2 | 4 | 7.5 | 4 | 6.0 |
| ND = not done | | | | | | |
| Blood obtained by cardiac puncture - 0.2 ml of blood placed into individual sequestrene bottles and blood counts done by Coulter counter. | | | | | | |

TABLE 8

| MEAN VALUES OF INDIVIDUAL ANIMALS PLATELETS x $10^{9}$/l | | | | | | |
|---|---|---|---|---|---|---|
| | N | Day 5 | N | Day 7 | N | Day 8 |
| CONTROLS | 2 | 632 | | ND | 2 | 649 |
| MITOZANTRONE | 3 | 631 | 4 | 845 | 4 | 1058 |
| MITOZANTRONE & CRONASSIAL® | 4 | 615 | 4 | 873 | 4 | 868 |
| ND = not done | | | | | | |
| Blood obtained by cardiac puncture - 0.2 ml of blood placed into individual sequenstrene bottles and blood counts done by Coulter counter. | | | | | | |

TABLE 9

| MEAN VALUES OF INDIVIDUAL ANIMALS WBC X $10^{9}$/l | | | | | | |
|---|---|---|---|---|---|---|
| | N | Day 5 | N | Day 7 | N | Day 8 |
| CONTROLS | 2 | 9.3 | | ND | 2 | 5.2 |
| MITOZANTRONE | 3 | 3.5 | 4 | 2.2 | 4 | 3.2 |
| MITOZANTRONE & CRONASSIAL® | 4 | 1.9 | 4 | 2.8 | 4 | 1.9 |
| ND = not done. | | | | | | |
| Blood obtained by cardiac puncture - 0.2 ml of blood placed into individual sequestrene bottles and blood counts done by Coulter counter | | | | | | |

A further series of experiments have also been carried out in order clearly to demonstrate the most effective time for administering mitozantrone and Cronassial® and in order to ascertain the toxicity reduction. The results set out in the following Tables 10 and 11 clearly show that there is a remarkable improvement when Cronassial® and mitozantrone are mixed together prior to injection in comparison with a separate but simultaneous administration.

TABLE 10

TOXICITY REDUCTION OF MITOZANTRONE DUE TO CRONASSIAL

| TUMOUR | HOST | SEX | TREATMENT | DAYS | ROUTE | TIMING | NO. DOSES | DOSE MG/KG | SURVIVORS d.30 | SURVIVAL (mean days) |
|--------|------|-----|-----------|------|-------|--------|-----------|------------|----------------|----------------------|
|  | SN | M | MTZ | 1.2.3 | iv |  | x 3 | 4 | 0/5 | 7.8 |
|  | SN | M | { MTZ CRON | 1.2.3 | iv. | mixed as one injection | x 3 | 4 200 | 4/5 | > 14 |

14

Similar experiments have been carried out using a combination of Cronassial® with adriamycin and mitomycin C. The results obtained with adriamycin are summarised in the following Tables 11 - 14 and with mitomycin C in Table 15.

In these Tables, MST means median survival time and T/C means treated/control.

TABLE 11

| DRUG | DOSE MG/KG | ANIMALS NO/GRP. | DEATHS | | SURVIVORS | | MST | T/C | COMMENTS |
|---|---|---|---|---|---|---|---|---|---|
| | | | TOXICITY | L1210 | 30d | 60d | | | |
| - | - | 8 | - | 8 | - | - | 6.8 | - | BDF ♀ 20g |
| ADR | 4.0 | 8 | - | 8 | - | - | 10.8 | 159 | |
| ADR CRON | 4.0 / 200 | 8 | 1 | 6 | - | 1 | 12.8 | >191 | |
| ADR | 6.0 | 8 | - | 8 | - | - | 9.7 | 135 | |
| ADR CRON | 6.0 / 200 | 8 | - | 8 | - | - | 12.1 | 180 | |
| ADR | 8.0 | 8 | - | 8 | - | - | 11.8 | 174 | |
| ADR CRON | 8.0 / 200 | 8 | - | 8 | - | - | 13.6 | 200 | |

BDF mice inoculated s.c. with L1210. All drugs or control solutions given i.p. in a vol. of 0.2 ml
All drugs given days 1, 2, 3.

15

TABLE 12

| DRUG | DOSE MG/KG | ANIMALS NO/GRP. | DEATHS | | SURVIVORS | | MST | T/C | COMMENTS |
|------|------------|-----------------|--------|--------|-----------|-----|-----|-----|----------|
| | | | TOXICITY | L1210 | 30d | 60d | | | |
| – | – | 8 | | 8 | – | – | 7.0 | – | |
| ADR | 6.0 | 8 | 7 | 1 | – | – | 9.38 | 134 | BDF ♂ 35g (6/12 old) |
| ADR ) CRON ) | 6.0 200 | 8 | 8 | – | – | – | 10.6 | 152 | |
| ADR | 8.0 | 8 | 8 | – | – | – | 5.8 | 83 | Toxic |
| ADR ) CRON ) | 8.0 200 | 8 | 7 | 1 | – | – | 8.4 | 120 | |
| ADR | 10.0 | 8 | 8 | – | – | – | 5.5 | 79 | ) Toxic |
| ADR ) CRON ) | 10.0 | 8 | 8 | – | – | – | 6.0 | 86 | ) |
| | | | | | | | | | |

BDF mice inoculated s.c. with L1210. All drugs or control solutions given i.p.
in a vol. of 0.2 ml
All drugs given days 1, 2, 3.

EP 0 345 251 B1

TABLE 13

| DRUG | DOSE MG/KG | ANIMALS NO/GRP. | DEATHS | | SURVIVORS | | MST | T/C | COMMENTS |
|---|---|---|---|---|---|---|---|---|---|
| | | | TOXICITY | L1210 | 30d | 60d | | | |
| - | - | 7 | - | 7 | - | - | 10.6 | | |
| ADR | 10.0 | 7 | 7 | - | - | - | 10.3 | 97 | BDF ♀ 25g (2-3/12 old |
| ADR CRON } | 10.0 200 | 7 | 7 | - | - | - | 16.43 | 155 | |
| ADR | 12.5 | 7 | 7 | - | - | -- | 5.4 | 51 | } Toxic |
| ADR CRON } | 12.5 200 | 7 | 7 | - | - | - | 5.3 | 50 | } |
| ADR | 15.0 | 7 | 7 | - | - | - | 5.1 | 48 | } Toxic |
| ADR CRON } | 15.0 | 7 | 7 | - | - | - | 6.0 | 57 | } |

BDF mice inoculated s.c. with L1210. All drugs or control solutions given i.p. in a vol. of 0.2 ml
All drugs given days 1, 2, 3.

DRUGS : ADR + CRN
TUMOUR: 3LL
PUPRPOSE: Metastases

TABLE 14

| DRUG | DOSE mg/kg | ANIMALS No/Grp. | $\bar{p}$ (mean weight) | $\bar{s}$ (mean no) | SURVIVORS | | MST | T/C | COMMENTS |
|---|---|---|---|---|---|---|---|---|---|
| | | | | | 30d | 60d | | | |
| CONTROL | – | 8 | 5.4g | 79.3 | 7 | – | – | – | Experiment terminated d21 |
| ADR | 3 | 8 | – | – | – | – | – | – | All ADR alone animals dead by d17 |
| ADR + CRN | 3 ) ) 200 ) | 8 | 4.7g | 26.7 | – | – | – | $\bar{p}$=0.9 $\bar{s}$=0.3 | All animals survived to d21 |

C57B1 mice·inoculated s.c. with Lewis lung carcinoma (3LL.)  All drugs or
control solutions given i.p. in a vol. of 0.2ml.
All drugs given days 6-10

EP 0 345 251 B1

DRUG : Mitomycin C (MMC)
TUMOUR: L1210
PURPOSE: CRN protection v. MMC toxicity

TABLE 15

| DRUG | DOSE mg/kg | ANIMALS No./Grp. | DEATHS | | SURVIVORS | | MST | T/C | COMMENTS |
|---|---|---|---|---|---|---|---|---|---|
| | | | TOXICITY | L1210 | 14d | 60d | | | |
| CONTROL | | 8 | 0 | 8 | - | - | 7.1 | | |
| MMC | 5 | 8 | 7 | | 1 | | 7.0 | | |
| MMC + CRN | 5) 200) | 8 | 3 | | 5 | | >14 | | |

BDF mice inoculated s.c. with L1210. All drugs or control solutions given i.p. in a vol. of 0.2ml.

All drugs given days 1, 2, 3.

**Claims**

**Claims for the following Contracting States : BE, CH, DE, FR, GB, IT, LI, LU, NL, SE**

1. Pharmaceutical composition for the treatment of cancer, comprising at least one anti-cancer drug selected from mitosene derivatives, anthracyclines, Vinca alkaloids and anthracenediones, in admixture with at least one ganglioside.

19

2. Pharmaceutical composition according to claim 1, wherein the anti-cancer drug is selected from mitomycin C, porfiromycin, adriamycin, vincristine and mitozantrone.

3. Pharmaceutical composition according to claim 1 or 2, wherein the ganglioside component is monosialotetraesosil ganglioside (GM-1) and/or disialotetraesosil ganglioside (GD-1a) and/or disialotetraesosil ganglioside (GD-1b) and/or trisialotetraesosil ganglioside (GT-1b).

4. Pharmaceutical composition according to any of the preceding claims, comprising a complex or reaction product of the anti-cancer drug and the ganglioside.

5. Pharmaceutical composition according to any of the preceding claims, whenever in the form of an isotonic solution for intravenous administration.

6. Pharmaceutical composition according to any of the preceding claims, wherein the weight ratio of anti-cancer drug to ganglioside is 1:1000, preferably 1:200 and more preferably 1:40.

**Claims for the following Contracting State : AT**

1. A process for preparing a pharmaceutical composition for the treatment of cancer, comprising at least one anti-cancer drug selected from mitosene derivatives, anthracyclines, Vinca alkaloids and anthracenediones, with at least one ganglioside, which comprises admixing the anti-cancer drug and the ganglioside.

2. The process according to claim 1, wherein the anti-cancer drug is selected from mitomycin C, porfiromycin, adriamycin, vincristine and mitozantrone.

3. The process according to claim 1 or 2, wherein the ganglioside component is monosialotetraesosil ganglioside (GM-1) and/or disialotetraesosil ganglioside (GD-1a) and/or disialotetraesosil ganglioside (GD-1b) and/or trisialotetraesosil ganglioside (GT-1b).

4. The process according to any of the preceding claims, wherein the composition comprises a complex or reaction product of the anti-cancer drug and the ganglioside.

5. The process according to any of the preceding claims, wherein the composition is in the form of an isotonic solution for intravenous administration.

6. The process according to any of the preceding claims, wherein the weight ratio of anti-cancer drug to ganglioside is 1:1000, preferably 1:200 and more preferably 1:40.

**Patentansprüche**
**Patentansprüche für folgende Vertragsstaaten : BE, CH, DE, FR, GB, IT, LI, LU, NL, SE**

1. Parmazeutisches Mittel zur Behandlung von Krebs, umfassend mindestens einen Anti-Krebs-Wirkstoff, ausgewählt unter Mitosenderivaten, Anthracyclinen, Vinca-Alkaloiden und Antracendionen, in Mischung mit mindestens einem Gangliosid.

2. Pharmazeutisches Mittel nach Anspruch 1, worin der Anti-Krebs-Wirkstoff ausgewählt ist unter Mitomycin C, Porfiromycin, Adriamycin, Vincristin und Mitozantron.

3. Pharmazeutisches Mittel nach Anspruch 1 oder 2, worin der Gangliosidteil Monosialotetraesosil-Gangliosid (GM-1) und/oder Disialotetraesosil-Gangliosid (GD-1a) und/oder Disialotetraesosil-Gangliosid (GD-1b) und/oder Trisialotetraesosil-Gangliosid (GT-1b) ist.

4. Pharmazeutisches Mittel nach einem der vorhergehenden Ansprüche, umfassend einen Komplex oder ein Reaktionsprodukt des Anti-Krebs-Wirkstoffes und des Gangliosids.

5. Pharmazeutisches Mittel nach einem der vorhergehenden Ansprüche in Form einer isotonischen Lösung zur intravenösen Verabreichung.

6. Pharmazeutisches Mittel nach einem der vorhergehenden Ansprüche, wobei das Gewichtsverhältnis des Anti-Krebs-Wirkstoffes zu dem Gangliosid 1:1000, vorzugsweise 1:200 und bevorzugter 1:40 ist.

**Patentansprüche für folgenden Vertragsstaat : AT**

1. Verfahren zur Herstellung eines parmazeutischen Mittels zur Behandlung von Krebs, umfassend mindestens einen Anti-Krebs-Wirkstoff, ausgewählt unter Mitosenderivaten, Anthracyclinen, Vinca-Alkaloiden und Antracendionen, in Mischung mit mindestens einem Gangliosid, wobei man den Anti-Krebs-Wirkstoff und das Gangliosid vermischt.

2. Verfahren nach Anspruch 1, worin der Anti-Krebs-Wirkstoff ausgewählt ist unter Mitomycin C, Porfiromycin, Adriamycin, Vincristin und Mitozantron.

3. Verfahren nach Anspruch 1 oder 2, worin der Gangliosidteil Monosialotetraesosil-Gangliosid (GM-1) und/oder Disialotetraesosil-Gangliosid (GD-1a) und/oder Disialotetraesosil-Gangliosid (GD-1b) und/oder Trisialotetraesosil-Gangliosid (GT-1b) ist.

4. Verfahren nach einem der vorhergehenden Ansprüche, worin das Mittel einen Komplex oder ein Reaktionsprodukt des Anti-Krebs-Wirkstoffes und des Gangliosids umfaßt.

5. Verfahren nach einem der vorhergehenden Ansprüche, worin das Mittel in Form einer isotonischen Lösung zur intravenösen Verabreichung vorliegt.

6. Verfahren nach einem der vorhergehenden Ansprüche, wobei das Gewichtsverhältnis des Anti-Krebs-Wirkstoffes zu dem Gangliosid 1:1000, vorzugsweise 1:200 und bevorzugter 1:40 ist.

**Revendications**
**Revendications pour les Etats contractants suivants : BE, CH, DE, FR, GB, IT, LI, LU, NL, SE**

1. Composition pharmaceutique pour le traitement du cancer, comprenant au moins un médicament anti-cancéreux choisi parmi les dérivés du mitosène, les anthracyclines, les alcaloïdes de Vinca et les anthracènediones, en mélange avec au moins un ganglioside.

2. Composition pharmaceutique selon la revendication 1, dans lequel le médicament anti-cancéreux est choisi parmi la mitomycine C, la porfiromycine, l'adriamycine, la vincristine et la mitozantrone.

3. Composition pharmaceutique selon la revendication 2, dans lequel le ganglioside est le ganglioside monosialotétraésosil (GM-1) et/ou le ganglioside disialotétraésosil (GD-1a) et/ou le ganglioside disialotétraésosil (GD-1b) et/ou le ganglioside trisialotétraésosil (GT-1b).

4. Composition pharmaceutique selon l'une quelconque des revendications précédentes, comprenant un complexe ou un produit de la réaction du médicament anti-cancéreux et du ganglioside.

5. Composition pharmaceutique selon l'une quelconque des revendications précédentes, sous forme d'une solution isotonique appropriée pour une administration par voie intraveineuse.

6. Composition pharmaceutique selon l'une quelconque des revendications précédentes, dans lequel le rapport pondéral du médicament anti-cancéreux/ganglioside est d'au moins 1/1000, de préférence 1/200 et plus avantageusement de 1/40.

**Revendication pour l'Etat contractant suivant : AT**

1. Un procédé de préparation d'une composition pharmaceutique pour le traitement du cancer, comprenant au moins un médicament anti-cancéreux choisi parmi les dérivés du mitosène, les anthracyclines, les alcaloïdes de Vinca et les anthracènediones, en mélange avec au moins un ganglioside, qui comprend le mélange du médicament anti-cancéreux et du ganglioside.

2. Le procédé selon la revendication 1, dans lequel le médicament anti-cancéreux est choisi parmi la

EP 0 345 251 B1

mitomycine C, la porfiromycine, l'adriamycine, la vincristine et la mitozantrone.

3. Le procédé selon la revendication 2, dans lequel le ganglioside est le ganglioside monosialotétraésosil (GM-1) et/ou le ganglioside disialotétraésosil (GD-1a) et/ou le ganglioside disialotétraésosil (GD-1b) et/ou le ganglioside trisialotétraésosil (GT-1b).

4. Le procédé selon l'une quelconque des revendications précédentes, dans lequel la composition comprend un complexe ou un produit de la réaction du médicament anti-cancéreux et du ganglioside.

5. Le procédé selon l'une quelconque des revendications précédentes, dans lequel la composition est sous forme d'une solution isotonique appropriée pour une administration par voie intraveineuse.

6. Le procédé selon l'une quelconque des revendications précédentes, dans lequel le rapport pondéral du médicament anti-cancéreux/ganglioside est d'au moins 1/1000, de préférence 1/200 et plus avantageusement de 1/40.

22

FIG.1
Adriamycin

# FIG.2

Adriamysin + Cronassial®

480    500    510    520    540    600    620    640    660

Drug "Uptake" after 1hr. circulation

FIG.3

Adriamycin + Cronassial®

Adriamycin

u/g. Adriamycin

EP 0 345 251 B1